(19) Europäisches Patentamt
European Patent Office
Office européen des brevets

(11) Publication number: **0 312 897 A1**

(12) # EUROPEAN PATENT APPLICATION

(21) Application number: 88116904.9

(22) Date of filing: 12.10.88

(51) Int. Cl.⁴: G01N 33/78

(30) Priority: 21.10.87 US 111748

(43) Date of publication of application:
26.04.89 Bulletin 89/17

(84) Designated Contracting States:
AT BE CH DE ES FR GB GR IT LI LU NL SE

(71) Applicant: ABBOTT LABORATORIES

Abbott Park Illinois 60064(US)

(72) Inventor: Green, Billy J.
425 Elmtree Lane
Vernon Hills Illinois 60061(US)
Inventor: Groskopf, William R.
1131 Dawes Avenue
Libertyville Illinois 60048(US)
Inventor: Kowal, Robert
15714 Birchwood Lane
Libertyville Illinois 60048(US)
Inventor: Larson, Thomas Alan
236 Southwick Court
Vernon Hills Illinois 60061(US)
Inventor: Wernke, Paul B., Jr.
6133 N. Austin Avenue
Chicago Illinois 60646(US)

(74) Representative: Modiano, Guido et al
MODIANO, JOSIF, PISANTY & STAUB
Modiano & Associati Baaderstrasse 3
D-8000 München 5(DE)

(54) **Thyroid hormone assay and reagent system employing furosemide.**

㊼ A method for measuring the concentration of a thyroid hormone selected from the group consisting of thyroxine ($T_4$) and triiodothyronine ($T_3$) in a sample comprising the steps of treating a sample with furosemide to displace $T_3$ and $T_4$ from binding proteins and measuring the amount of $T_3$ and $T_4$ displaced. A thyroid hormone reagent system is also disclosed to measure the concentration of thyroxine ($T_4$) and triiodothyronine ($T_3$) in a sample comprising furosemide in an amount sufficient to substantially displace all $T_3$ and $T_4$ bound to binding proteins in a sample.

FIG. 1

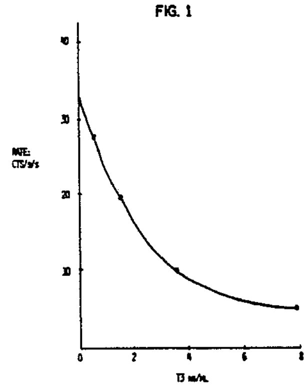

EP 0 312 897 A1

## Thyroid Hormone Assay and Reagent System Employing Furosemide

The present invention is directed toward a method for measuring thyroid hormones such as thyroxine ($T_4$) and triiodothyronine ($T_3$) in biological samples. The method comprises the use of furosemide (4-chloro-N-(2-furylmethyl)-5-sulfamoylanthranilic acid) or analogs thereof to displace the thyroid hormones from the binding proteins such that the thyroid hormones can be measured with suitable assay reagents. Furosemide has the advantage of not inhibiting the binding of the thyroid hormones or tracers to the antibody employed in the assay because it has very low crossreactivity with antibody.

The measurement of $T_3$ and $T_4$ concentrations in biological samples such as plasma and serum is very valuable in the diagnosis and treatment of various diseases and physiological conditions. In normal human serum, about 99.97% of $T_4$ and 99.7% of $T_3$ present, is bound to proteins such as thyroxine-binding globulin (TBG), prealbumin (TBPA) and albumin; the remaining 0.03% of $T_4$ and 0.3% of $T_3$ is present as unbound (dialyzable or free) hormones. The serum concentration of thyroid hormones, along with other factors such as thyroidal secretion and degradation, is also dependent on serum concentration of TBG; other binding proteins in serum are relatively less important since their binding affinity for thyroid hormones is far less than that of TBG. The concentration of TBG is elevated in conditions such as pregnancy, estrogen treatment or a genetic abnormality; serum total $T_4$ and $T_3$ is increased in these situations. Conversely, serum TBG concentration is decreased during treatment with androgens or due to a genetic deficiency of TBG. This is associated with decreased concentrations of thyroid hormones. However, in either of the above-mentioned situations of altered TBG concentration, the patient can be euthyroid and concentrations of free $T_4$ and $T_3$ and that of total $T_4$ and $T_3$ corrected for TBG abnormality are within the range of normal subjects.

U.S. Patent 3,911,096 discloses a radioimmunoassay method for determining $T_3$ or $T_4$ concentrations in human serum samples. The method incubates serum sample with barbital or other buffers to displace $T_3$ or $T_4$ with a "blocking agent", radioactive labeled $T_3$ or $T_4$ and antibody specific for $T_3$ or $T_4$. The amount of bound radioactive $T_3$ or $T_4$ is then measured to determine hormone concentration against a standardized curve. The "blocking agents" can include 8-anilino-1-napthalene-sulfonic acid ·ANS),3-(4-anilino-1-napthylazo)2,7-napthalene disulfonic acid (ANNDS),2,4,6-trinitro benzene sulfonic acid (TNBS), napthalene sulfonic acid, Thimerosal, 5,5-diphenyl 2-thiohydantain, Doxepin HCl, Diazepam, sodium salicylate, prochlorperazine, halofenate (MK-185) Merck, Sharpe and Dohme.

More recent methods do not use barbital or buffers but instead only employ the so called "blocking agents" as described in U.S. Patent 3,911,096 such as ANS. A disadvantage of ANS is that it is light sensitive which can interfere with absorption measurements.

Older methods used solvent extraction, enzymic digestion of the sample or other agents to extract the $T_3$ or $T_4$ hormone. These methods have proved to be cumbersome and the treatment steps have interfered with the accurate determination of the thyroid hormone concentrations. Therefore an improved method for the determination of $T_3$ and $T_4$ hormones is desirable.

### Summary of the Invention

The present invention is directed toward a method for measuring the concentration of a thyroid hormone selected from the group consisting of thyroxine ($T_4$) and triiodothyronine ($T_3$) in a sample comprising the treatment of a sample with furosemide to displace $T_3$ and $T_4$ from binding proteins and measuring the amount of $T_3$ and $T_4$ displaced. The amount of $T_3$ and $T_4$ displaced is measured using any of a variety of known thyroid hormone assay reagents and methods. Preferably, the furosemide replaces any other displacing agent or "popper" and displacing steps formerly employed in a conventional reagent system or method.

The amount of furosemide used is calculated to displace substantially all $T_3$ and $T_4$ as a function of sample size. The method of detection can be fluorescence, absorption, fluorescence polarization, chemiluminescence or radioactive means.

In another aspect, the present invention is directed toward a reagent system designed to measure the concentration of a thyroid hormone selected from the group consisting of thyroxine ($T_4$) and triiodothyronine ($T_3$) in a sample comprising furosemide in an amount sufficient to substantially displace all $T_3$ and $T_4$ bound to binding proteins in a sample.

The remainder of the reagent system can be any of a variety of thyroid reagent systems. Preferably,

the furosemide replaces all the conventional displacing agent or "popper" or displacing step generally employed in such a thyroid reagent system. The reagent systems and methodologies can comprise fluorescent enzyme-immunoassay, fluorescent polarization immunoassay, chemiluminescence immunoassay, radioimmunoassay and colorimetric enzyme-immunoassay to name a few.

## Detailed Description of the Invention

A method of determining thyroxine ($T_4$) and triiodothyronine ($T_3$) in biological samples such as whole blood, serum and plasma is described. The method uses furosemide as a displacing agent in immunoassays for the thyroid hormones. It displaces the hormones from their protein binders such as thyroxine-binding globulin (TBG), albumin, and prealbumin such that the direct measurement of these hormones in serum or plasma can be made with an appropriate antibody.

Furosemide is particularly advantageous as a displacing agent because it does not interfere with the binding of the analyte $T_3$ or $T_4$ or the tracer (i.e., $T_3$ or $T_4$ derivatives such as enzyme, enzyme inhibitors, fluorescent, or radioactive labels) to the antibody. This is an advantage over other classical displacing agents such as 8-anilino-1-napthalene-sulfonic acid (ANS) or salicylate. Further, furosemide retains its solubility in the assay reagent system which improves its handling characteristics.

Furosemide is directly added to the sample with the reagent used to identify the free thyroid hormone. It can be employed in any thyroid reagent system such as those designed for fluorescence, absorption, chemiluminescence or radioimmunoassays. Thus, furosemide is a convenient additive for a thyroid assay reagent system as a displacing agent or "popper". More preferably, furosemide can be employed to completely eliminate any other displacing agent or hormone displacing steps formerly employed in an otherwise conventional thyroid assay reagent system and method. The quantity of furosemide employed is dependent on the amount required to displace the hormone from the binding protein(s) under the conditions of the particular assay. This can easily be determined by those skilled in the use of thyroid hormone assays as a function of available sample to be tested.

Furosemide (4-chloro-N-(2-furylmethyl)-5-sulfamoylanthranilic acid) can be represented by the structural formula as follows:

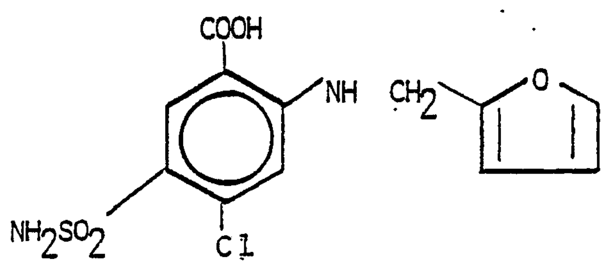

Analogs or derivatives of furosemide which can produce similar results as a displacing agent for a thyroid reagent system and are therefore contemplated to be within the scope of this invention.

The method of the subject invention uses furosemide as the displacing agent for the thyroid hormones in any of a variety of thyroid reagent system. The furosemide is added to the sample in an amount sufficient to displace substantially all, preferably all, the available bound thyroid hormone ($T_3$ and $T_4$). The sample can be either treated with furosemide before adding the reagent system chosen to quantitate the released thyroid hormone or it can be added simultaneously with the reagent system.

The furosemide displacing agent can be employed in any of a variety of thyroid reagent systems to quantitate the thyroid hormone displaced by either competitive or noncompetitive assay techniques. Also the furosemide supplemented reagent system can employed with any of a variety of detection methods such as fluorescence, absorption, fluorescence polarization, chemiluminescence, or radioactivity. Typical labels used in thyroid hormone assays and compatible with furosemide can include fluorescent enzyme labels, chemilumenescent labels, colorimetric labels, luminenscent labeled materials, radioactive labels and metal or nonmetal labels.

The following examples illustrate the use of furosemide as a displacing agent in the measurement of thyroid hormones.

EXAMPLE I

This example uses furosemide as a displacing agent in the measurement of thyroid hormones and illustrates the making of a standard curve, to allow accurate correlation of unknown $T_4$ concentrations with a standard curve.

The reagents employed in this enzyme inhibitor immunoassay were acetylcholinesterase, furosemide and a $T_4$-binding antiserum or $T_4$ antibody obtained from a sheep immunized with thyroxine conjugated to bovine thyroglobulin. The $T_4$ antibody was used in a final dilution of 1:60,000. The steps of the enzyme inhibitor immunoassay procedure were performed as described below.

A first aqueous reagent consisted of 16.6 microliters per liter (uL/L) of sheep antithyroxine antiserum, 34 units per liter (U/L) of acetylcholinesterase and 0.7 millimoles per liter (mM/L) of furosemide in a Hepes buffer with protein stabilizer (0.6 moles per liter lithium sulfate).

A second aqueous reagent consisted of 8 nanomoles per liter (nM/L) of thyroxine-inhibitor conjugate, 2 mM/L of 5,5′-dithiobis-(2-nitrobenzoic acid) (DTNB) and 3.85 mM/L of S-acetyl-B-(methylthio)choline iodide in a citrate buffer.

The first reagent solution was added to 3.6 microliters of plasma in an amount of 168 microliters and mixed. This mixture was allowed to incubate for approximately five minutes. Next, 85 microliters of the second reagent solution was added and absorbance readings taken at 415 and 500 nanometer wavelengths at defined time intervals.

In this thyroid hormone assay, sample thyroxine competes with an enzyme inhibitor ($T_4$-conjugate), which is structurally similar to thyroxine, for binding sites on a specific antibody. The amount of free conjugate is determined by measuring the rate at which enzyme activity is lost. $T_4$-conjugate and specimen thyroxine are bound in proportion to their respective concentrations in solution. Furosemide is used to release thyroxine from serum binding proteins and prevent subsequent binding.

This test utilized 5 calibrators with nominal amounts of thyroxine at the following concentrations 0, 3, 6, 12 and 24 micrograms per deciliter (ug/dL). The time constant for disappearance of enzyme activity is fit to a 4 parameter logistic curve to generate a calibration curve. The concentration range of the thyroxine calibrators (0-24 ug/dL) is adequate to accurately determine the thyroxine concentration in most patient samples encountered. When samples containing thyroxine in concentrations greater than the highest calibrator (24 ug/dL) are encountered they can be accurately determined by diluting the sample with the calibrator and reassaying. The sample thyroxine value is calculated by multiplying the result from the diluted sample by the dilution factor.

EXAMPLE II

This example uses furosemide as a displacing agent in the measurement of thyroid hormones in an enzyme-immunoassay. The reagents employed were as follows:

$T_3$ binding antiserum bound to polystyrene particles obtained from a goat immunized with a covalent conjugate of $T_3$ and bovine thyroglobulin. The antibody coated particles are diluted such that the proper dose response is obtained in the assay. The furosemide is present in this reagent at a concentration of 0.0125% (weight/volume).

A $T_3$-alkaline phosphatase conjugate was prepared and diluted in buffer containing protein stabilizers.

Reagent grade Na-L-$T_3$ was added gravimetrically to $T_3$ free human serum to a final concentration of 0, 0.5, 1, 2, 4 and 8 nanograms per milliliter (ng/ml). These materials served as calibrators.

The substrate consisted of an aqueous buffered solution of 4-methylumbelliferoyl phosphate. The assay diluent consisted of 50 millimolar of Tris, 0.3 molar NaCl, pH 7.5.

The following steps were carried out for this assay.

Fifty microliters (uL) of calibrator or sample was added to a polystyrene reaction well. To this was added 50 uL of particle reagent followed by 150 uL of assay diluent. This mixture was mixed and incubated for approximately ten minutes at 34.5° C. The furosemide in the particle reagent effectively displaced the $T_3$ from binding protein.

A 175 uL volume of the mixture was transferred to a glass fiber matrix and washed with two 75 uL aliquots of assay diluent. The particles to which released $T_3$ was bound were irreversibly bound to the matrix.

The $T_3$ alkaline phosphate conjugate reagent was added to the matrix and after a four minute incubation unreacted conjugate was washed from the matrix with two 75 uL aliquots of assay diluent.

Substrate was then added to the matrix and the rate of formation of product, methyl umbelliferone, was

determined by fluorescent emission.

Figure 1 shows a typical standard curve plotted as rate in counts per second per second (CTS/s/s) versus the amount of bound $T_3$ measured in nanograms per milliliter (ng/ml). The fifty percent intercept is 2.0 ng/ml and the sensitivity of the assay is measured by its ability to distinguish a sample from zero with 95% confidence in 0.13 ng/ml. The plot indicates the excellent results obtained with the thyroid reagent system containing furosemide.

Table 1 illustrates $T_3$ values obtained in the assay of Example II (EIA) compared to a typical radioimmunoassay (RIA) which utilizes 8-anilino-1-naphthalene-sulfonic acid (ANS) as a deblocking agent.

TABLE I

| Sample | EIA with furosemide | RIA with ANS |
|---|---|---|
| low $T_3$ | 0.42 ± 0.20 | 0.51 ± 0.18 |
| normal $T_3$ | 1.53 ± 0.25 | 1.60 ± 0.31 |
| hyperthyroid | 4.19 ± 1.36 | 4.20 ± 1.24 |

The results indicate that using furosemide as a displacing agent yields valid sample values in that the assay is able to distinguish low, normal and high $T_3$ samples, i.e., it is as effective as classical methodologies in displacing $T_3$ from its binding proteins.

## Claims

1. A method for measuring the concentration of a thyroid hormone selected from the group consisting of thyroxine ($T_4$) and triiodothyronine ($T_3$) in a sample comprising:
   a) treating a sample with furosemide to displace $T_3$ and $T_4$ from binding proteins;
   b) measuring amount of $T_3$ and $T_4$ displaced.

2. The method of Claim 1 wherein the amount of furosemide is calculated to displace substantially all $T_3$ and $T_4$ as a function of sample size.

3. The method of Claim 2 wherein said step (b) is conducted by fluorescence, absorption, fluorescence polarization, chemiluminescence or radioactive means.

4. The method of Claim 1 wherein said sample is a plasma or serum sample.

5. A reagent system designed to measure the concentration of a thyroid hormone selected from the group consisting of thyroxine ($T_4$) and triiodothyronine ($T_3$) in a sample comprising:
furosemide in an amount sufficient to substantially displace all $T_3$ and $T_4$ bound to binding proteins in a sample.

6. The reagent system of Claim 5 which is fluorescent enzyme-immunoassay.

7. The reagent system of Claim 5 which is a chemiluminescence immunoassay.

8. The reagent system of Claim 5 which is a radioimmunoassay.

9. The reagent system of Claim 5 which is a colorimetric enzyme-immunoassay.

FIG. 1

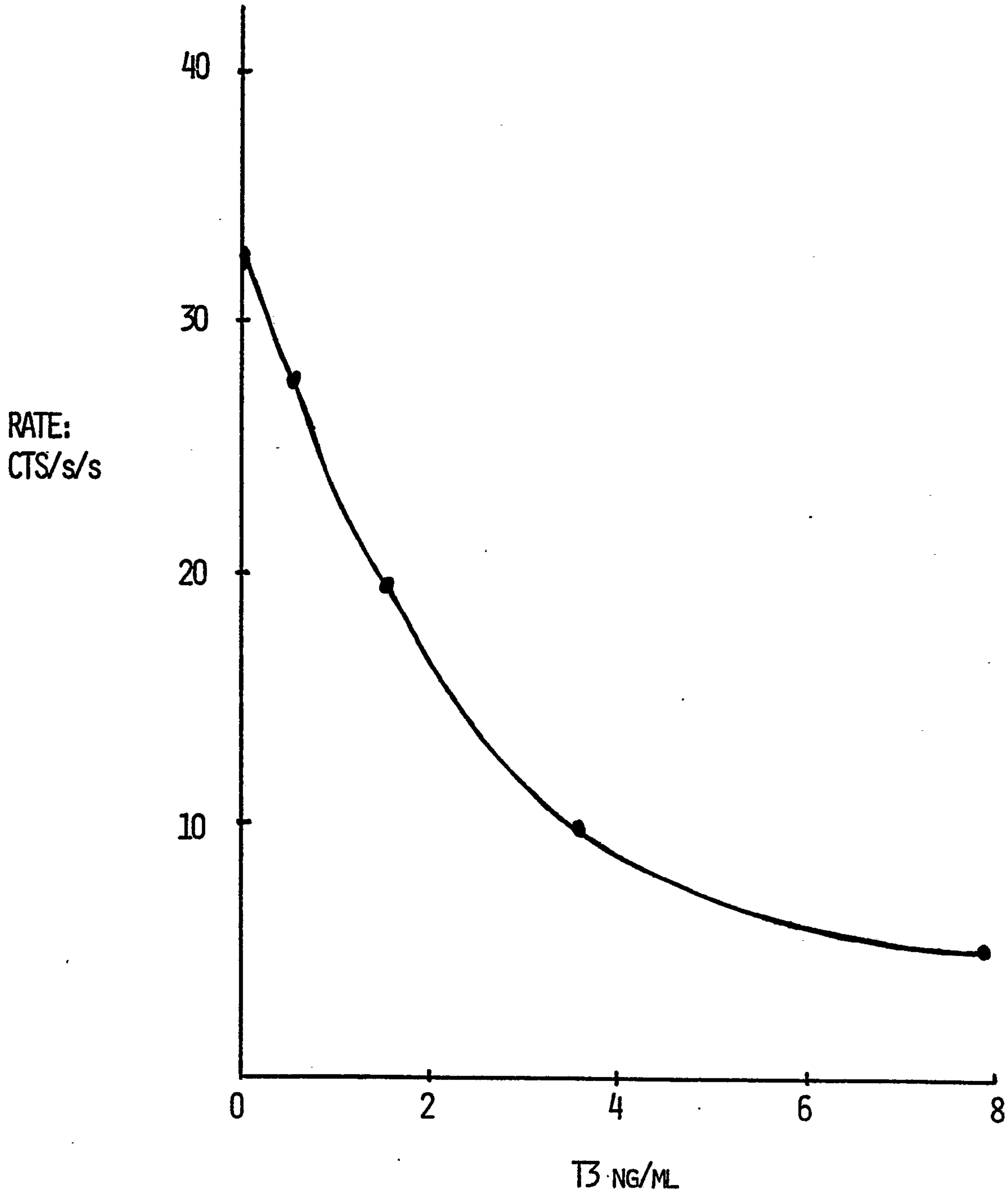

European Patent Office

# EUROPEAN SEARCH REPORT

Application Number

EP 88 11 6904

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CLINICAL CHEMISTRY, vol. 33, no. 10, 1987, pages 1752-1755, Winston-Salem, US; S. BENVENGA et al.: "Effect of free fatty acids and nonlipid inhibitors of thyroid hormone binding in the immunoradiometric assay of thyroxin-binding globulin" * Whole article * --- | 1-9 | G 01 N 33/78 |
| Y | CHEMICAL ABSTRACTS, vol. 103, no. 1, 8th July 1985, page 14. column 1, abstract no. 156a, Columbus, Ohio, US; J.R. STOCKIGT et al.: "Interaction of furosemide with serum thyroxine-binding sites: in vivo and vitro studies and comparison with others inhibitors", & J. CLIN. ENDOCRINOL. METAB. 1985, 60(5), 1025-31 * Abstract * --- | 1-9 | |
| Y | CHEMICAL ABSTRACTS, vol. 105, no. 13, 29th September 1986, page 42, column 1, abstract no. 108184p, Columbus, Ohio, US; Y. SADANAGA et al.: "Effect of furosemide on serum thyroid hormone determination in vitro", & EISEI KENSA 1986, 35(4), 635-8 * Abstract * --- | 1-9 | TECHNICAL FIELDS SEARCHED (Int. Cl.4) |
| | | | G 01 N |
| Y | EP-A-0 078 477 (MILES LABORATORIES, INC.) * Whole document * --- | 1-9 | |
| Y | EP-A-0 133 464 (MILES LABORATORIES, INC.) * Whole document * --- -/- | 1-9 | |

The present search report has been drawn up for all claims

| Place of search | Date of completion of the search | Examiner |
|---|---|---|
| THE HAGUE | 01-02-1989 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 88 11 6904

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 155 104 (AMERSHAM INTERNATIONAL PLC)<br>* Whole document *<br>--- | 1-9 | |
| A | CHEMICAL ABSTRACTS, vol. 101, no. 9, 27th August 1984, pages 113-114, column 2, abstract no. 66557x, Columbus, Ohio, US; J.R. STOCKIGT et al.: "High concentrations of furosemide inhibit serum binding of thyroxine", & J. CLIN. ENDOCRINOL. METAB. 1984, 59(1), 62-6<br>--- | | |
| D,A | US-A-3 911 096 (I.J. CHOPRA)<br>----- | | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 01-02-1989 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document